# EUROPÄISCHE PATENTSCHRIFT

(11) **EP 2 710 988 B1**
(45) Veröffentlichungstag und Bekanntmachung des Hinweises auf die Patenterteilung: **04.11.2015**
(21) Anmeldenummer: 13182939.2
(22) Anmeldetag: 04.09.2013
(51) Int. Cl.: A61F 5/01, A61F 5/14, A43B 7/26

(54) **Schuheinlage**
Shoe inlay
Semelle de chaussure

(30) Priorität: 25.09.2012 DE 202012009229 U; 17.01.2013 DE 102013000730
(43) Veröffentlichungstag der Anmeldung: 26.03.2014
(73) Patentinhaber: Mueller, Tamara, 73466 Lauchheim (DE)
(72) Erfinder: Mueller, Alexander, 73466 Lauchheim (DE)
(74) Vertreter: Schmid, Wolfgang

(56) Entgegenhaltungen:
- EP-A2- 1 454 544
- US-A- 1 554 883
- US-A- 2 075 552

## Beschreibung

Die Erfindung betrifft eine Schuheinlage mit einem Grundelement und mit wenigstens einem mit dem Grundelement verbundenen, aus der Ebene des Grundelements hervorstehenden Zehentrennsteg.

Eine derartige Schuheinlage ist beispielsweise aus der EP 1 090 612 B1 bekannt. Mit solchen Einlagen soll eine Zehenfehlstellung des Menschen korrigiert werden. Beispiele für solche Zehenfehlstellungen sind unter den Fachbegriffen Hallux Valgus oder Digitus Varus bekannt. Alternativ zu den Schuheinlagen werden häufig auch Operationen oder sonstige Eingriffe am Fuß durchgeführt, um die Zehenfehlstellung zu beseitigen.

Keine der bekannten Vorgehensweisen berücksichtigt dabei jedoch die Dynamik des Fußes beim Laufen und somit im Alltag. Aufgrund der Dynamik des Fußes wirken Reibungs- und Scherkräfte auf das Gewebe und die Knochenstruktur des Fußes und können daher die Beweglichkeit aller betroffenen Gelenke und Strukturen beeinträchtigen und schädigen, was zu nicht unerheblichen Verletzungen führen kann.

Aus der US 1,554,883 A1 ist eine weitere Schuheinlage bekannt, bei der ein verstellbarer Zehentrennsteg vorgesehen ist. Die Verstellung des Zehentrennstegs erfolgt durch eine Verschiebung desselben in Querrichtung der Schuheinlage, so dass der Zehentrennsteg je nach Position desselben zwischen unterschiedlichen Zehen angeordnet werden kann.

Einen ähnlichen Stand der Technik, bei dem ein in Querrichtung der Schuheinlage verstellbarer Zehentrennsteg vorgesehen ist, ist in der US 2,075,552 A1 beschrieben.

Aus der EP 1 454 544 A2 ist eine Schuheinlage für Babyschuhe bekannt, bei der zwischen dem ersten und dem zweiten Zeh ein starrer Trennsteg angeordnet ist.

Es ist daher Aufgabe der vorliegenden Erfindung, eine Schuheinlage zu schaffen, die die physiologischen Voraussetzungen des menschlichen Fußes besser berücksichtigt.

Erfindungsgemäß wird diese Aufgabe durch die in Anspruch 1 genannten Merkmale gelöst.

Durch den erfindungsgemäßen Zehentrennsteg wird eine Berührung zweier benachbarter Zehen, zwischen denen sich der Zehentrennsteg befindet, unterbunden, wodurch ein Aneinanderreiben dieser Zehen beim Laufen verhindert und damit Druckstellen oder gar Hämatome vermieden werden. Die erfindungsgemäße Verschieblichkeit des Trennstegs ermöglicht dabei eine Anpassung desselben an unterschiedlichste Füße, da beim Anziehen eines mit der erfindungsgemäßen Schuheinlage ausgestatteten Schuhs der Zehentrennsteg durch das sich zwischen den Zehen befindende Gewebe verschoben wird. Dadurch wird vermieden, dass durch den Zehentrennsteg ein zu großer Druck auf das Gewebe ausgeübt wird, wodurch wiederum Druckstellen bzw. Hämatome vermieden werden.

Durch den verschieblichen und damit dynamischen Zehentrennsteg werden außerdem die benachbarten Zehen in ihre natürliche Stellung gebracht und dort gehalten. Da der Zehentrennsteg nach distal und proximal verschieblich ist, werden Druck-, Reibungs- und Scherkräfte vermieden, die in sämtlichen Schrittphasen auftreten können.

Des Weiteren wird durch den erfindungsgemäßen Zehentrennsteg die Position des Hallux aus der Valgus-Stellung in seine physiologisch richtige Stellung geführt. Durch dieses Korrigieren des Hallux aus seiner Valgus-Stellung werden sämtliche Muskelgruppen des Fußes, insbesondere des Hallux, aktiviert und stimuliert.

Ein weiterer Vorteil des verschieblichen Zehentrennstegs besteht darin, dass dieser nicht fest mit dem Grundelement der Schuheinlage verbunden ist und somit ausgewechselt und erneuert werden kann, beispielsweise wenn der Zehentrennsteg beschädigt ist oder wenn ein geometrisch anderer Zehentrennsteg eingebaut werden soll. Auf diese Weise wird die Flexibilität der Schuheinlage erheblich erhöht, da für physiologisch unterschiedliche Füße unterschiedliche Zehentrennstege eingesetzt werden können.

Um den Zehentrennsteg sicher mit dem Grundelement verbinden zu können, kann in einer vorteilhaften Weiterbildung der Erfindung vorgesehen sein, dass das Grundelement einen Durchbruch zum Durchführen des Zehentrennstegs aufweist.

Wenn in einer weiteren vorteilhaften Ausgestaltung der Erfindung der Zehentrennsteg eine Grundplatte und ein an der Grundplatte angebrachtes, über die Ebene des Grundelements hervorstehendes Trennelement aufweist, so ist durch die Grundplatte ein Halt des Zehentrennstegs in dem Grundelement möglich, wobei das an dem Grundelement angebrachte bzw. einteilig mit demselben ausgeführte Trennelement die Funktionalität des Zehentrennstegs sicherstellt.

Um die erfindungsgemäße Verschieblichkeit des Zehentrennstegs gegenüber dem Grundelement auf einfache Weise zu realisieren, kann vorgesehen sein, dass die Grundplatte in einer Führung des Grundelements verschieblich gelagert ist.

Eine sehr einfache und zuverlässige Möglichkeit zur Bildung der Führung innerhalb des Grundelements ergibt sich, wenn das Grundelement mehrere Schichten aufweist, wobei die Führung durch eine Aussparung in wenigstens einer der Schichten gebildet ist.

Eine weitere vorteilhafte Ausgestaltung der erfindungsgemäßen Lösung kann darin bestehen, dass der wenigstens eine Zehentrennsteg bei bestimmungsgemäßem Gebrauch der Schuheinlage zwischen der ersten und der zweiten Zehe angeordnet ist. Mit einem derart angeordneten Zehentrennsteg lässt sich die Zehenfehlstellung Hallux Valgus des großen Zehs sehr einfach korrigieren.

Eine weitere Ausgestaltung der Erfindung kann darin bestehen, dass der wenigstens eine Zehentrennsteg bei bestimmungsgemäßem Gebrauch der Schuheinlage zwischen der vierten und der fünften Zehe angeordnet ist. Mit einem solchen Zehentrennsteg wird die als Digitus Varus bekannte Zehenfehlstellung des kleinen Zehs korrigiert.

Des Weiteren kann vorgesehen sein, dass das Grundelement eine bei bestimmungsgemäßem Gebrauch unterhalb des Grundgelenks der ersten Zehe angeordnete Erhöhung aufweist. Eine solche Erhöhung, die insbesondere von medial nach lateral abfallend geformt sein kann und durch deren Keilform ein Drehmoment in das Grundelement der ersten Zehe eingebracht wird, ergibt sich eine Stützung und Korrektur des Grundgelenks der ersten Zehe, die der Zehenfehlstellung Hallux Valgus entgegenwirkt. Hierzu kann die Erhöhung in Abhängigkeit der jeweiligen Fehlstellung der Zehen in geeigneter Weise positioniert werden.

Wenn in einer weiteren vorteilhaften Ausgestaltung der Erfindung das Grundelement eine bei bestimmungsgemäßem Gebrauch unter dem Grundgelenk der fünften Zehe angeordnete Erhöhung aufweist, so ergibt sich eine Korrektur der fünften bzw. kleinen Zehe, die der Zehenfehlstellung Digitus Varus entgegenwirkt.

Durch die erfindungsgemäße Schuheinlage können also die unterschiedlichsten Zehenfehlstellungen korrigiert werden, indem an den jeweiligen Fuß angepasste Einlagen gefertigt werden, bei denen sowohl der Zehentrennsteg als auch die gegebenenfalls vorgesehenen Erhöhungen an der jeweils erforderlichen Stelle positioniert werden können. Die Erhöhungen unterstützen dabei den Zehentrennsteg durch Supination und Pronation sowohl des Grundgelenks als auch des Apex der ersten Zehe.

Nachfolgend ist ein Ausführungsbeispiel der Erfindung anhand der Zeichnung prinzipmäßig dargestellt.

Es zeigt:
- Fig. 1: eine Draufsicht auf eine erfindungsgemäße Schuheinlage;
- Fig. 2: einen Schnitt nach der Linie II-II aus Fig. 1;
- Fig. 3: einen Schnitt nach der Linie III-III aus Fig. 1;
- Fig. 4: eine Ansicht gemäß dem Pfeil IV aus Fig. 1; und
- Fig. 5: eine Ansicht gemäß dem Pfeil V aus Fig. 1.

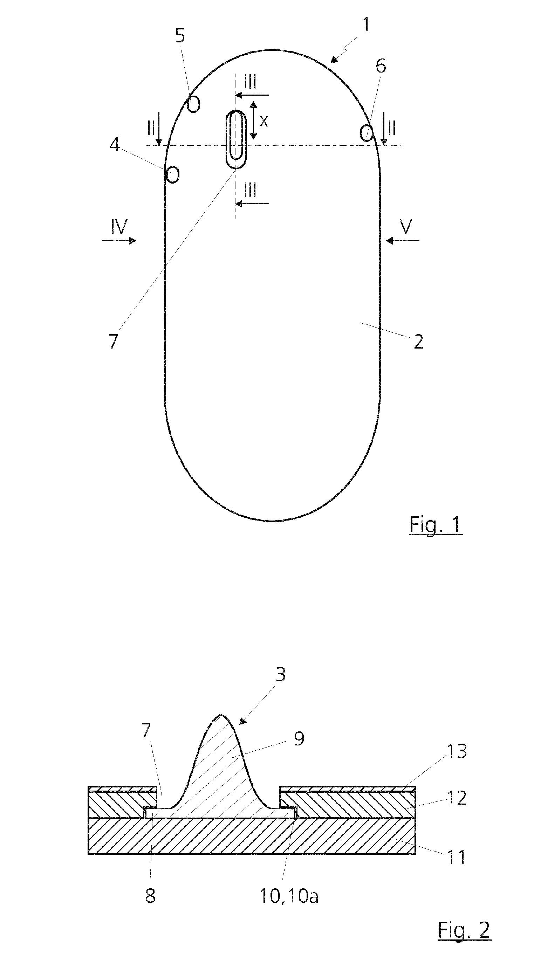

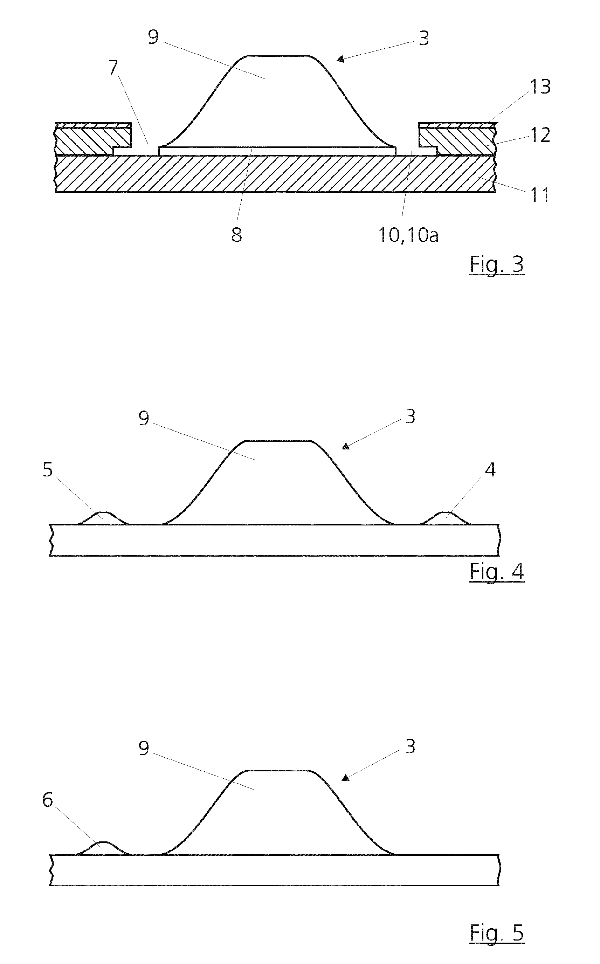

Fig. 1 zeigt eine sehr schematische Draufsicht auf eine Schuheinlage 1, die ein Grundelement 2 und einen mit dem Grundelement 2 verbundenen, aus der Ebene des Grundelements 2 hervorstehenden Zehentrennsteg 3 aufweist. Das Hervorstehen des Zehentrennstegs 3 aus der Grundebene der Schuheinlage 1 ist in den Schnitten von Fig. 2 und Fig. 3 sowie in den Ansichten der Figuren 4 und 5 deutlich erkennbar.

Der Zehentrennsteg 3 ist gegenüber dem Grundelement 2 in der mit "x" bezeichneten Richtung verschieblich, so dass sich bei der Benutzung der Schuheinlage 1 der Zehentrennsteg 3 verschiebt, wenn ein Benutzer mit dem sich zwischen zwei Zehen befindenden Gewebe an dem Zehentrennsteg 3 anstößt.

Während beim dargestellten Ausführungsbeispiel der Zehentrennsteg 3 zwischen der ersten bzw. großen und der zweiten Zehe angeordnet ist, ist es auch möglich, den Zehentrennsteg 3 zwischen zwei beliebigen anderen Zehen anzuordnen, beispielsweise zwischen der vierten und der fünften bzw. kleinen Zehe. Grundsätzlich kann sich der Zehentrennsteg 3 zwischen zwei beliebigen Zehen, also an insgesamt vier unterschiedlichen Positionen, befinden. Des Weiteren ist es auch möglich, mehr als einen Zehentrennsteg 3 vorzusehen. Theoretisch könnte demnach zwischen jeder Zehe ein Zehentrennsteg 3 vorgesehen sein, d. h. vier Zehentrennstege 3. Zusätzlich zu dem wenigstens einen verschieblichen Zehentrennsteg 3 könnte grundsätzlich auch ein oder mehrere starre, nicht dargestellte Zehentrennstege vorgesehen sein.

Die Schuheinlage 1 weist des Weiteren mehrere Erhöhungen, nämlich eine bei bestimmungsgemäßen Gebrauch der Schuheinlage 1 unterhalb des Grundgelenks der ersten Zehe angeordnete erste Erhöhung 4, eine bei bestimmungsgemäßem Gebrauch unterhalb des vorderen Gelenks der ersten Zehe angeordnete zweite Erhöhung 5 und eine bei bestimmungsgemäßem Gebrauch unterhalb des Grundgelenks der fünften bzw. kleinen Zehe angeordnete Erhöhung 6 auf. Die Erhöhungen 4, 5 und 6 sind in den Seitenansichten der Figuren 4 und 5 nochmals dargestellt und es wird aus der Zusammenschau der Figuren 1, 4 und 5 deutlich, dass die Erhöhungen 4, 5 und 6 von medial nach lateral abfallend geformt sind. Die Erhöhungen 4, 5 und 6 sind also in Richtung der Innenseite der Schuheinlage 1 vertieft und in Richtung des äußeren Rands derselben erhöht. Es ist nicht erforderlich, sämtliche der Erhöhungen 4, 5 und 6 an ein und derselben Schuheinlage 1 vorzusehen. Vielmehr hat jede der Erhöhungen 4, 5 und 6 ihre eigene, spezifische Wirkung. Dennoch können sich aus der gemeinsamen Verwendung mehrerer der Erhöhungen 4, 5 und 6 Synergieeffekte ergeben.

Die Erhöhungen 4, 5 und 6 können als separate, beispielsweise durch Verkleben mit dem Grundelement 2 verbundene Bauteile ausgeführt sein. Es ist jedoch auch möglich, die Erhöhungen 4, 5 und 6 bei der vorzugsweise durch Fräsen erfolgenden Herstellung des Grundelements 2 in dasselbe einzubringen bzw. an demselben vorzusehen. Die erstgenannte Möglichkeit ist insbesondere dann sinnvoll bzw. wünschenswert, wenn die Erhöhungen 4, 5 und/oder 6 an einem bereits bestehenden Grundelement 2 angebracht werden sollen, wohingegen die zweitgenannte Möglichkeit dann zum Einsatz kommt, wenn ein neues Grundelement 2 für eine neue Schuheinlage 1 hergestellt wird.

Die beiden Erhöhungen 4 und 5 gleichen die Biegung des Hallux nach außen aus. Gegebenenfalls kann auch lediglich eine der beiden Erhöhungen 4 oder 5 eingesetzt werden. Durch den Abstand der beiden Erhöhungen 4 und 5 voneinander und die dadurch entstehende Vertiefung wird verhindert, dass in einem Bereich zwischen den Gelenken eine derartige Kraft auf den Hallux aufgebracht wird, dass dadurch möglicherweise Schmerzen entstehen können. Zusätzlich wird durch die Erhöhungen 4, 5 und 6 die Muskeltätigkeit der jeweils betroffenen Bereiche des Fußes erhöht. Des Weiteren werden durch die Erhöhungen 4, 5 und 6 die betreffenden Gelenke entlastet. Die Erhöhungen 4, 5 und 6 könnten auch andere als die dargestellten geometrischen Formen aufweisen. Insbesondere ergibt sich die Form sowie die Position der einzelnen Erhöhungen 4, 5 oder 6 aus der Form des Gelenks und der Stellung der jeweiligen Zehe.

Um den Zehentrennsteg 3 mit dem Grundelement 2 der Schuheinlage 1 zu verbinden, weist das Grundelement 2 im vorliegenden Fall einen in der Draufsicht von Fig. 1, jedoch auch in den Schnitten der Figuren 2 und 3 erkennbaren Durchbruch 7 auf, der in Form einer Aussparung in dem Grundelement 2 ausgebildet ist.

Aus den Schnittansichten der Figuren 2 und 3 ist erkennbar, dass der Zehentrennsteg 3 eine Grundplatte 8 und ein an der Grundplatte 8 angebrachtes, über die Ebene des Grundelements 2 hervorstehendes Trennelement 9 aufweist. Das Trennelement 9 ist dasjenige Element des Zehentrennstegs 3, das die Trennwirkung des Zehentrennstegs 3 erzeugt, also die Wirkung, dass zwei benachbarte Zehen durch den Zehentrennsteg 3 auseinandergehalten werden. Dagegen wird durch die Grundplatte 8 die verschiebliche Anbringung des Zehentrennstegs 3 an dem Grundelement 2 der Schuheinlage 1 bewirkt.

Des Weiteren ist in den Schnittansichten der Figuren 2 und 3 erkennbar, dass die Grundplatte 8 des Zehentrennstegs 3 in einer Führung 10 des Grundelements 2 verschieblich gelagert ist. Hierbei ist die Führung 10 durch eine Aussparung 10a in einer von mehreren Schichten des Grundelements 2, nämlich einer unteren Schicht 11, einer mittleren Schicht 12 und einer oberen Schicht 13 gebildet. Im vorliegenden Fall ist die Aussparung 10a in der mittleren Schicht 12 vorgesehen und die Breite der Aussparung 10a ist, wie aus Fig. 2 hervorgeht, etwas größer als die Breite der Grundplatte 8 des Zehentrennstegs 3, so dass der Zehentrennsteg 3 sehr einfach entlang der Führung 10 verschoben werden kann. Die Länge der Aussparung 10a ist, wie sich aus dem Schnitt von Fig. 3 ergibt, deutlich größer als die Länge der Grundplatte 8, so dass die Verschieblichkeit des Zehentrennstegs 3 entlang der Führung 10 sichergestellt ist.

Das Grundelement 2 ist also in Sandwichbauweise hergestellt und es ist möglich, den Zehentrennsteg 3 von dem Grundelement 2 zu demontieren und gegebenenfalls durch einen neuen oder einen anderen zu ersetzen. Die obere Schicht 13 ist dabei als Deckschicht ausgeführt und kann aus den unterschiedlichsten Materialien bestehen, um im Hinblick auf die Bedürfnisse und Verträglichkeiten der jeweiligen Nutzer so wenig Einschränkungen wie möglich zu erzeugen.

Selbstverständlich kann die Schuheinlage 1 in beliebigen Schuhgrößen hergestellt werden. Auch der Zehentrennsteg 3 sowie die gegebenenfalls vorgesehenen Erhöhungen 4, 5 und 6 können in beliebigen Größen und Anordnungen ausgeführt sein, um eine Anpassung der Schuheinlage 1 an die jeweiligen physiologischen Gegebenheiten zu ermöglichen.

Dadurch, dass sich die Aussparung 10a in der mittleren Schicht 12 befindet, wird trotz des durch den Fuß auf die Schuheinlage 1 und damit auch auf die Grundplatte 8 des Zehentrennstegs 3 wirkenden Kraft ein Einklemmen des Zehentrennstegs 3 verhindert. Durch die Aussparung 10a wird außerdem verhindert, dass durch die Höhe der Grundplatte 8 die Gesamthöhe des Grundelements 2 vergrößert wird.

In nicht dargestellter Weise ist es auch möglich, an der Schuheinlage 1 einen oder mehrere Riemen anzubringen, um diesen in der Art eines Schuhs, in diesem Fall einer Sandale, auszuführen. Im Bereich der Zehen könnte in diesem Fall eine Schutzkappe oder ähnliches vorgesehen sein, so dass sich ein halbgeschlossener Schuh ergeben könnte.

## Patentansprüche

1. Schuheinlage mit einem Grundelement und mit wenigstens einem mit dem Grundelement verbundenen, aus der Ebene des Grundelements hervorstehenden Zehentrennsteg,
**dadurch gekennzeichnet, dass**
der wenigstens eine Zehentrennsteg (3) nach distal und proximal gegenüber dem Grundelement (2) verschieblich ist.

2. Schuheinlage nach Anspruch 1,
**dadurch gekennzeichnet, dass**
das Grundelement (2) einen Durchbruch (7) zum Durchführen des Zehentrennstegs (3) aufweist.

3. Schuheinlage nach Anspruch 1 oder 2,
**dadurch gekennzeichnet, dass**
der Zehentrennsteg (3) eine Grundplatte (8) und ein an der Grundplatte (8) angebrachtes, über die Ebene des Grundelements (2) hervorstehendes Trennelement (9) aufweist.

4. Schuheinlage nach Anspruch 3,
**dadurch gekennzeichnet, dass**
die Grundplatte (8) in einer Führung (10) des Grundelements (2) verschieblich gelagert ist.

5. Schuheinlage nach Anspruch 4,
**dadurch gekennzeichnet, dass**
das Grundelement (2) mehrere Schichten (11,12,13) aufweist, wobei die Führung (10) durch eine Aussparung (10a) in wenigstens einer der Schichten (11,12,13) gebildet ist.

6. Schuheinlage nach einem der Ansprüche 1 bis 5,
**dadurch gekennzeichnet, dass**
der wenigstens eine Zehentrennsteg (3) bei bestimmungsgemäßem Gebrauch der Schuheinlage (1) zwischen der ersten und der zweiten Zehe angeordnet ist.

7. Schuheinlage nach einem der Ansprüche 1 bis 6,
**dadurch gekennzeichnet, dass**
der wenigstens eine Zehentrennsteg (3) bei bestimmungsgemäßem Gebrauch der Schuheinlage (1) zwischen der vierten und der fünften Zehe angeordnet ist.

8. Schuheinlage nach einem der Ansprüche 1 bis 7,
**dadurch gekennzeichnet, dass**
das Grundelement (2) eine bei bestimmungsgemäßem Gebrauch unter einem vorderen Gelenk der ersten Zehe angeordnete Erhöhung (5) aufweist.

9. Schuheinlage nach einem der Ansprüche 1 bis 8,
**dadurch gekennzeichnet, dass**
das Grundelement (2) eine bei bestimmungsgemäßem Gebrauch unter dem Grundgelenk der fünften Zehe angeordnete Erhöhung (6) aufweist.

## Claims

1. Shoe insole having a base element and having at least one toe-separating web connected to the base element and projecting from the plane of the base element,
**characterized in that**
the at least one toe-separating web (3) is displaceable distally and proximally with respect to the base element (2).

2. Shoe insole according to Claim 1,
**characterized in that**
the base element (2) has an aperture (7) for guiding through the toe-separating web (3).

3. Shoe insole according to Claim 1 or 2,
**characterized in that**
the toe-separating web (3) has a baseplate (8) and a separating element (9) which is mounted on the baseplate (8) and projects over the plane of the base element (2).

4. Shoe insole according to Claim 3,
**characterized in that**
the baseplate (8) is displaceably mounted in a guide (10) of the base element (2).

5. Shoe insole according to Claim 4,
**characterized in that**
the base element (2) has a plurality of layers (11, 12, 13), wherein the guide (10) is formed by a cutout (10a) in at least one of the layers (11, 12, 13).

6. Shoe insole according to one of Claims 1 to 5,
**characterized in that**,
during correct use of the shoe insole (1), the at least one toe-separating web (3) is arranged between the first and the second toe.

7. Shoe insole according to one of Claims 1 to 6,
**characterized in that**,
during correct use of the shoe insole (1), the at least one toe-separating web (3) is arranged between the fourth and the fifth toe.

8. Shoe insole according to one of Claims 1 to 7,
**characterized in that**
the base element (2) has an elevation (5) which, during correct use, is arranged below an anterior joint of the first toe.

9. Shoe insole according to one of Claims 1 to 8,
**characterized in that**
the base element (2) has an elevation (6) which, during correct use, is arranged below the metatarsophalangeal joint of the fifth toe.

## Revendications

1. Semelle de chaussure comportant un élément de base et au moins une arête liée à l'élément de base et protubérant par rapport à l'élément de base,
**caractérisée en ce que**,
ladite au moins une arête (3) est coulissante distalement et proximalement par rapport à l'élément de base (2).

2. Semelle de chaussure selon la revendication 1,
**caractérisée en ce que**,
l'élément de base (2) comporte une cavité (7) pour permettre la traversée de l'arête (3).

3. Semelle de chaussure selon la revendication 1 ou 2,
**caractérisée en ce que**,
l'arête (3) comporte une plaque de base (8) et un élément de séparation (9) monté sur la plaque de base (8) et en protubérance par rapport à l'élément de base (2).

4. Semelle de chaussure selon la revendication 3,
**caractérisée en ce que**,
la plaque de base (8) peut être bloquée dans un guidage (10) ménagé dans l'élément de base (2).

5. Semelle de chaussure selon la revendication 4,
**caractérisée en ce que**,
l'élément de base (2) comprend plusieurs couches (11, 12, 13), dans laquelle le guidage (10) est formé par un évidement (10a) ménagé dans au moins une des couches (11, 12, 13).

6. Semelle de chaussure selon l'une des revendications 1 à 5,
**caractérisée en ce que**,
ladite au moins une arête (3) est disposée, entre le premier et le deuxième doigt de pied, lors d'une utilisation sur ordonnance de la semelle (1), comme support orthopédique.

7. Semelle de chaussure selon l'une des revendications 1 à 6,
**caractérisée en ce que**,
ladite au moins une arête (3) est disposée, entre le quatrième et le cinquième doigt de pied, lors d'une utilisation sur ordonnance de la semelle (1), comme support orthopédique.

8. Semelle de chaussure selon l'une des revendications 1 à 7,
**caractérisée en ce que**,
l'élément de base (2) comporte un rehaussement (5) disposé sous une articulation des premiers doigts de pied, lors d'une utilisation sur ordonnance comme support orthopédique.

9. Semelle de chaussure selon l'une des revendications 1 à 8,
**caractérisée en ce que**,
l'élément de base (2) comporte un rehaussement (6) disposé sous l'articulation de base des cinquièmes doigts de pied, lors d'une utilisation sur ordonnance.
